# EUROPEAN PATENT APPLICATION

(11) **EP 4 331 586 A1**
(43) Date of publication of application: **06.03.2024**
(21) Application number: 22193333.6
(22) Date of filing: 31.08.2022
(51) Int. Cl.: A61K 31/5377, A61K 31/616, A61P 9/10, A61K 9/24

(54) **ANTICOAGULANT THERAPY WITH AN IMPROVED DOSAGE REGIMEN**

(71) Applicant: Adamed Pharma S.A., 05-152 Czosnow (PL)
(72) Inventor: Berdzik-Kalarus, Sylwia, 40-534 Katowice (PL); Owczarz-Rabiega, Malgorzata, 95-200 Pabianice (PL)
(74) Representative: Matusiewicz, Katarzyna Joanna

(57) **Abstract**

The present invention relates to an oral fixed-dose immediate release pharmaceutical composition of 2.5 mg of rivaroxaban and 50 mg of acetylsalicylic acid for use in a prevention of atherothrombotic events in adult patients after an acute coronary syndrome (ACS) with elevated cardiac biomarkers or for the prevention of atherothrombotic events in adult patients with coronary artery disease (CAD) and/or symptomatic peripheral artery disease (PAD) at high risk of ischaemic events.

## Description

The need to improve the effectiveness of anticoagulant therapy in secondary prevention is indicated by the fact that intensive antiplatelet therapy only partially reduces cardiovascular risk.

Effectiveness of the proposed therapies and dosage regimens may and they usefulness to be accepted by regulatory agencies and consequently safely applied in human medicine can be assesses on the base of appropriate trials.

COMPASS trail results deliver evidence that additional blockage of selected levels of the clotting cascade may have a significant impact on the subsequent one prevention of serious cardiovascular events in a selected population of patients.

COMPASS trials show a consistent and significant reduction of the composite of major adverse cardiac and limb vascular events comparing patients treated with the combination of 2.5 mg rivaroxaban plus Acetylsalicylic acid to Acetylsalicylic acid alone, across a broad spectrum of PAD patients.

In the COMPASS study a dosage regimen of 2.5 mg dosage of rivaroxaban twice daily accompanied by 100 mg Acetylsalicylic acid administered once daily was proved to be superior to rivaroxaban 5mg twice daily as well as over Acetylsalicylic acid 100 mg once daily.

Based on these results, a new dosage regimen has been approved for prevention of atherothrombotic events in adult patients after an acute coronary syndrome (ACS) and in adult CAD/PAD patients, which are advised to take 2.5 mg of rivaroxaban twice daily and a daily dose of 75-100 mg of Acetylsalicylic acid.

However, the only clinically tested dosage of Acetylsalicylic acid was 100 mg, which was administered once daily.

In the US 10828310 B2 different combinations of rivaroxaban dosages with Acetylsalicylic acid dosages are presented in form of lists of options to be combined to form suitable combination therapy options. In this document is stated there that, an effective reduction of the risk of stroke, myocardial infarction, or cardiovascular death, in a human patient with peripheral arterial disease is achieved when rivaroxaban is administered in an amount of 2.5 mg twice daily and Acetylsalicylic acid is administered in an amount of 75-100 mg daily.

In the description of US 10828310 B2 it is mentioned that in one embodiment the twice daily rivaroxaban dosage (selected form a list) is foreseen in combination with an Acetylsalicylic acid dosage (selected form a list) administered twice daily.

In one section of this description, the twice daily rivaroxaban dosage is in combination with Acetylsalicylic acid administered twice daily. The dosages are to be selected from the respective lists. The twice daily Acetylsalicylic acid dosages being 30, 35, 40, 45, 50, 51, 52, 53, 54, 55, 56, 57, 58, 59, 60, 61, 62, 63, 64, 65, 66, 67, 68, 69, 70, 71 , 72, 73, 74, 75, 76, 77, 78, 79, 80, 81, 82, 83, 84, 85, 86, 87, 88, 89, 90, 91, 50 92, 93, 94, 95, 96, 97, 98, 99, 100, 101, 102, 103, 104, 105, 106, 107, 108, 109, 110, 115, 120, 125, 130, 135, 140, 145, or 150 mg and rivaroxaban dosages are to be selected from the following values: 0.5, 0.6, 0.7, 0.8, 0.9, 1.0, 1.1, 1.2, 1.3, 1.4, 1.5, 1.6, 1.7, 1.8, 1.9, 2.0, 2.1 , 2.2, 2.3, 2.4, 2.5, 2.6, 2.7, 2.8, 2.9, 3.0, 3.1 , 3.2, 3.3, 3.5, or 3.5 mg.

It is also mentioned in US 10828310 B2 that a combination therapy of rivaroxaban and Acetylsalicylic acid is understood such as rivaroxaban and Acetylsalicylic acid are provided in one product. In claim 5 a specific administration regimen is provided as once daily administration of a first product comprising rivaroxaban and Acetylsalicylic acid and a second product comprising rivaroxaban, wherein the first product comprises 2.5 mg rivaroxaban and 75-100 mg Acetylsalicylic acid.

With this it is clear that the dosage regiment foreseen be the prior art is following: administration of 2.5 mg of rivaroxaban twice daily accompanied by Acetylsalicylic acid dosage form the range 75-100 mg taken at any time during the day, or administration of
2.5 mg of rivaroxaban once daily accompanied by Acetylsalicylic acid dosage form the range 75-100 mg taken at the same time.

However, particular dosages of Acetylsalicylic acid in such a regimen are not disclosed.

Combination of the disclosure of US 10828310 with the fact that only the 100 mg dosage of Acetylsalicylic acid to be taken once daily combined with 2.5 mg dosage of rivaroxaban taken twice daily finds a clinical substantiation doesn't provide a support for a product in form of a fixed dose combination for uniform application of rivaroxaban and Acetylsalicylic acid dosages and thus uniform levels of both drugs over a day.

### Problem to be solved

Therefore, there still remains a need for improved fixed-dose pharmaceutical composition of rivaroxaban and Acetylsalicylic acid, which will provide better patients compliance and more consistent platelet inhibition and consequently improved effectiveness of anticoagulant combination therapy.

There still also remains a need for fixed-dose pharmaceutical composition of rivaroxaban and Acetylsalicylic acid for use in a prevention of atherothrombotic events in adult patients after an acute coronary syndrome (ACS) with elevated cardiac biomarkers or for the prevention of atherothrombotic events in adult patients with coronary artery disease (CAD) or symptomatic peripheral artery disease (PAD) at high risk of ischaemic events.

There also remains a need for an improved oral dosage regimen for reducing the risk of myocardial infarction, stroke or cardiovascular death in a human patient with coronary artery disease and/or peripheral artery disease, of rivaroxaban and acetylsalicylic acid.

There also remains a need for an improved oral dosage regimen with the use of fixed-dose pharmaceutical composition of rivaroxaban and Acetylsalicylic acid.

### Solution of the problem

The present invention solves to the above-mentioned problem by provision of
a fixed-dose pharmaceutical composition for oral administration comprising
component a) comprising 2.5 mg of rivaroxaban and
component b) comprising 50 mg of Acetylsalicylic acid,
and pharmaceutically acceptable excipients.

Fixed-dose composition is understood as composition is a single dosage-form. The dosage form is an immediate release form, i.e. both component a) and component b) are in a form of immediate release, so that the dosage-form containing component a) and component b) form disintegrates rapidly and get dissolved to release the Rivaroxaban and Acetylsalicylic acid without any modification. It is intended that excipients, diluents or carriers do not prolong the rate of release and/or absorption of Rivaroxaban and Acetylsalicylic acid.

In particular, the present invention relates to the following aspects:
(1) According to an aspect of the present invention, a fixed-dose pharmaceutical composition for oral administration is provided,
   the composition comprising:
   component a) comprising 2.5 mg of rivaroxaban and one or more
   pharmaceutically acceptable excipients; and
   component b) comprising 50 mg of Acetylsalicylic acid and one or more
   pharmaceutically acceptable excipients, and
   one or more pharmaceutically acceptable excipients.
(2) The pharmaceutical composition according to (1), wherein the pharmaceutical
   composition is a single dosage form in the form of a tablet, in which the
   component a) forms a first layer, the component b) forms a second layer, and
   the first layer at least partially covers the surface of the second layer. Preferably, the tablet is a bilayer tablet.
(3) The pharmaceutical composition according to (1) or (2), wherein the pharmaceutical
   composition is a single dosage form in the form of a tablet, in which the
   component a) forms a first layer, the component b) forms a second layer, and
   the first layer completely covers the surface of the second layer. In this aspect the component b) can form a first layer while the component a) forms a second layer.
(4) The pharmaceutical composition according to (3), wherein the tablet is uncoated or
   coated. Preferably, the tablet is uncoated. Also preferably the tablet is coated.
(5) The pharmaceutical composition according to (1), wherein the pharmaceutical
   composition is a single dosage form in the form of a coated tablet, in which the component b) forms a core of the tablet and component a) is in a form of a coat applied on this core.
(6) The pharmaceutical composition according to (5), wherein further coat is applied on the coat formed by component a).
(7) The pharmaceutical composition according to (5) or (6), wherein an intermediate coat is applied on the component b), between the tablet core and the coat with the component a).
(8) The pharmaceutical composition according to (1), wherein the pharmaceutical composition is a single dosage form in the form of a hard capsule, in which the
   and component a) is in a form of a granulate or a pellet or a tablet, preferably a pellet or a tablet, most preferably a tablet and component b) is in a form of a granulate or a pellet or a tablet, preferably a pellet or a tablet, most preferably a tablet.
(9) The pharmaceutical composition according to (8), wherein both the component a) and the component b) is in form of a tablet.
(10) The pharmaceutical composition according to any one of (1) to (19), wherein the
   component a) comprises, as the at least one excipient, at least one of a filler, a disintegrant, a binder, a lubricant, a glidant and any combination thereof. Preferably the component a) comprises a filler, a disintegrant, a binder, a lubricant, and, optionally, a glidant. More preferably, the component a) contains a binder in addition to the filler, the disintegrant, the and the lubricant.
(11) The pharmaceutical composition according to (1) to (10), wherein the component b) comprises, as the at least one excipient, at least one of a filler, a disintegrant, a binder, a lubricant, a glidant and any combination thereof. Preferably, the component b) comprises a filler, a disintegrant, a glidant, a lubricant, and, optionally, a binder. More preferably, the component b) contains no binder.
(12) The pharmaceutical composition according to any one of (1) to (11), wherein the component a) and/or the component b) comprise a filler. Preferably, the component a) and the component b) comprise a filler.
(13) The pharmaceutical composition according to (14), wherein the filler is at least one independently selected from the group consisting of anhydrous or monohydrate lactose, sucrose, microcrystalline cellulose, starch such as maize starch, pregelatinized starch, microcrystalline cellulose coated with colloidal silica, mannitol, sorbitol and any combination thereof, preferably the filler is at least one independently selected from the group consisting of anhydrous or monohydrate lactose, mannitol, microcrystalline cellulose, maize starch, saccharose and any combination thereof, and more preferably the filler is at least one selected from the group consisting of lactose monohydrate, microcrystalline cellulose, or a combination thereof.
(14) The pharmaceutical composition according to (12) or (13), wherein the immediate release component a) contains the filler in an amount of 5 to 90 wt.%, preferably 10 to 60 wt.%, also preferably 25 to 50 wt. %, based on the weight of the component a), and/or wherein the component b) contains the filler in an amount of 5 to 90 wt.%, preferably 10 to 60 wt.%, also preferably 25 to 50 wt. %, based on the weight of the component b).
(15) The pharmaceutical composition according to any one of (1) to (15), wherein the component a) and/or the component b) comprise a binder.

Preferably, the component a) comprises a binder.
(16) The pharmaceutical composition according to (19), wherein the binder is at least one independently selected from the group consisting of hydroxypropylcellulose,
   hydroxypropylmethylcellulose, methylcellulose, carboxymethylcellulose, hydroxycellulose, hydroxyethylcellulose, polyvinyl alcohol, polyvinylpyrrolidone, copovidone, pregelatinized starch, gelatine and any combination thereof, preferably the binder is at least one independently selected from the group consisting of hydroxypropyl cellulose, polyvinylpyrrolidone and any combination thereof, and more preferably the binder is Hypromellose, more specifically Hypromellose E 5 with viscosity: 4-6 mPa s.
(17) The pharmaceutical composition according to (15) or (16), wherein the
   component a) contains the binder in an amount of 0.4 to 10 wt.%, preferably 0.5 wt.%,
   based on the weight of the component a), and/or wherein the component b) contains the binder in an amount of 0.4 to 5 wt.%, preferably 0.7 or 0.9 wt.%, based on the weight of the component b). Preferably, only component a) contains a binder.
(18) The pharmaceutical composition according to any one of (1) to (17), wherein the component a) and/or the component b) comprise a disintegrant. Preferably, the component a) and the component b) comprise a disintegrant.
(19) The pharmaceutical composition according to (18), wherein the disintegrant is at least one independently selected from the group consisting of carboxymethylcellulose sodium salt,
   sodium croscarmellose, microcrystalline cellulose, crospovidone, sodium starch glycolate,
   maize starch, and any combination thereof, preferably the disintegrant is at least one independently selected from the group consisting of sodium starch glycolate, sodium
   croscarmellose, maize starch and any combination thereof, more preferably the disintegrant is sodium croscarmellose or sodium starch glycolate.
(20) The pharmaceutical composition according to (18) or (19), wherein component a) contains the disintegrant in an amount of 0.5 to 25 wt.%, preferably 2 to 16 wt.%, more preferably 5 wt.%, based on the weight of the component a), and/or wherein the component b) contains the disintegrant in an amount of 0.3 to 5 wt.%, preferably 2 to 5 wt.%, more preferably 3 wt.%, based on the weight of the component b).
(21) The pharmaceutical composition according to any one of (1) to (20), wherein the
   component a) and/or the component b) comprise a lubricant. Preferably, the component a) and the component b) comprise a lubricant.
(22) The pharmaceutical composition according to (21), wherein the lubricant is at least one independently selected from the group consisting of stearic acid, magnesium stearate, calcium stearate, sodium stearyl fumarate, glycerol tristearate, palm oil derivatives such as palmitic acid or hydrogenated palm oil, hydrogenated vegetable oil, such as hydrogenated castor oil, and any combination thereof, preferably the lubricant is at least one independently selected from magnesium stearate and stearic acid.
(23) The pharmaceutical composition according to (21) or (22), wherein the
   component a) contains the lubricant in an amount of 0.1 to 5 wt.%, preferably 2.3, 0.45 or 0.5 wt.%, based on the weight of the component a), and/or wherein the component b) contains the lubricant in an amount of 0.1 to 5 wt.%, preferably 0.45 wt.%, based on the weight of the component b).
(24) The pharmaceutical composition according to any one of (1) to (23), wherein the component a) and/or the component b) comprise a glidant.

Preferably, the component b) but not the component a) comprises a glidant.
(25) The pharmaceutical composition according to (24), wherein the glidant is at least one independently selected from the group consisting of fumed silica (colloidal silicon dioxide),
   such as colloidal anhydrous silica, starch, talc and any combination thereof, preferably the glidant is colloidal anhydrous silica.
(26) The pharmaceutical composition according to (24) or (25), wherein the immediate
   release component a) contains the glidant in an amount of 0.1 to 5.0 wt.%, preferably 1.6 or 1.0 or 0.9 wt.%, based on the weight of the component b), and/or wherein the component b) contains the glidant in an amount of 0.1 to 5.0 wt.%, preferably 0.1 to 1.0 wt.%, more preferably up to 0.9 wt.%, based on the weight of the component b).
(27) The pharmaceutical composition according to any one of (1) to (26), wherein the
   component a) comprises 5 to 90 wt.% of a filler, 0.5 to 25 wt.% of a disintegrant, 0.1 to 5.0 wt.% of a lubricant, 0.4 to 10 wt.% of a binder, and optionally 0.1 to 5.0 wt.% of a glidant based on the total weight of the component a).
(28) The pharmaceutical composition according to any one of (1) to (27), wherein the
   component a) comprises 0.4 to 10 wt.%, preferably 0.4 to 3 wt.%, more preferably 0.7 wt.%, of a binder, based on the total weight of the component a).
(29) The pharmaceutical composition according to any one of (1) to (28), wherein the
   component b) comprises 5 to 90 wt.% of a filler, 0.5 to 5 wt.% of a disintegrant, 0.1 to 5.0 wt.% of a lubricant, 0.1 to 5.0 wt.% of a glidant, and optionally 0.4 to 5 wt.% of a binder, based on the total weight of the component b).
(30) The pharmaceutical composition according to any one of (1) to (7) and (10) to (28), wherein the component a) comprises 3-8 wt.% of a standard coating system based on the total weight of the component a) wherein the rest constitutes rivaroxaban.
(30) The pharmaceutical composition according to any one of (1) to (31), wherein the component b) comprises 5 to 90 wt.% of a filler, 0.5 to 5 wt.% of a disintegrant, 0.1 to 3.0 wt.% of a lubricant, 0.1 to 1.0 wt.% of a glidant, and optionally 0.4 to 5 wt.% of a binder, based on the total weight of the component b).
(31) The pharmaceutical composition according to any one of (1) to (30), wherein the component a) has a rivaroxaban content of 1 to 5 wt.%, preferably 1.5 to 4.5 wt.%, more preferably 2.6 to 3.1 wt.%, such as 3 wt.%, based on the total weight of the component a); furthermore, the component b) has an acetylsalicylic acid content of 20 to 80 wt.%, preferably 35 to 75 wt.%, more preferably 33 to 72 wt.%, such as 70, 65, 60, 55 or 55.5 or 55.6 wt.%, based on the total weight of the component b).
(32) The pharmaceutical composition according to any one of (1) to (31), wherein the component a) has a rivaroxaban content of 0.2 to 5 wt.%, preferably 0.35 to 2.5 wt.%, more preferably 0.5 to 1.5 wt.%, such as 0.55 wt.%, based on the total weight of the film-coated tablet and, the component b) has an acetylsalicylic acid content of 5 to 25 wt.%, preferably 10 to 20 wt.%, more preferably 11 to 15 wt.%, such as 11.2 wt.%, based on the total weight of the film-coated tablet.
(33) The pharmaceutical composition according to any one of (1) to (32), wherein the component a) has a rivaroxaban content of 0.5 to 5 wt.%, preferably 0.8 to 2.5 wt.%, more
   preferably 1 to 1.5 wt.%, such as 1.1 wt.%, based on the total weight of the bilayer tablet and the component b) has an acetylsalicylic acid content of 10 to 50 wt.%, preferably 15 to 35 wt.%, more preferably 20 to 25 wt.%, such as 22.7 wt.%, based on the total weight of the bilayer tablet.
(34) The pharmaceutical composition according to any one of (1) to (33), wherein the
   component a) contains 3.0 wt.% of rivaroxaban, 53.5 wt.% of microcrystalline cellulose, 5.0 wt.% of croscarmellose sodium, 30.0 wt.% of Lactose monohydrate, 2.0 wt.% of hypromellose, 2.0 wt.% of sodium lauryl sulphate and 1.0 wt.% of magnesium stearate, based on the total weight of the component a); and component b) contains 55.6 wt. % of acetylsalicylic acid, 28.3 wt. % of cellulose microcrystalline, 14.4 wt. % of starch, maize, 1.1 wt. % of silica, colloidal anhydrous and 0.6 wt. % stearic acid based on the total weight of component b).
(35) The pharmaceutical composition according to any one of (1) to (34), wherein the component a) contains 0.5 wt. % of rivaroxaban and 9.6 wt.% of a coating system based on the total weight of the component a) and the component b) contains 11.2 wt. % of acetylsalicylic acid, 51.7 wt. % of cellulose microcrystalline, 25.6 wt. % of starch, maize, 0.9 wt. % of silica, colloidal anhydrous and 0.45 wt. % stearic acid based on the total weight of component b)
(36) The pharmaceutical composition according to any one of (1) to (35), wherein the
   component a) contains 1.1 wt. % of rivaroxaban, 11.4 wt. % of lactose monohydrate, 0.2 wt. % of cellulose microcrystalline, 1.8 wt. % of croscarmellose sodium, 0.7 wt. % of hypromellose, 0.7 wt. % of sodium lauryl sulphate and 0.4 wt. % of magnesium stearate based on the total weight of the component a) and the component b) contains 22.7 wt. % of acetylsalicylic acid, 26.6 wt. % of cellulose microcrystalline, 13.4 wt. % of starch, maize, 0.6 wt. % of silica, colloidal anhydrous and 0.4 wt. % stearic acid based on the total weight of the component b)
(37) The pharmaceutical composition according to any one of (1) to (7) and (10) to (36) wherein the component a) contains 2.5mg of rivaroxaban, and 3-8 wt. % of standard coating system based on the total weight of the pharmaceutical composition and the component b) contains 11.2 wt. % of acetylsalicylic acid, 26.6 wt. % of cellulose microcrystalline, 13.4 wt. % of starch, maize, 0.6 wt. % of silica, colloidal anhydrous and 0.4 wt. % stearic acid based on the total weight of the component b).
(38) The pharmaceutical composition according to any one of (1) to (37), wherein the
   pharmaceutical composition is a single dosage form and provides, after oral administration, a maximum plasma concentration of rivaroxaban (Cmax) of 77.69±28.2 ng/mL after median tₘₐₓ of 2 hours after administration, and an area under the plasma concentration time curve from time zero to infinity of 481.26±22.3 ng·h/mL, as determined by human PK studies.
(39) The pharmaceutical composition according to any one of (1) to (38), wherein the pharmaceutical composition provides, after oral administration of a single dosage form of the pharmaceutical composition twice a day and preferably 12 hours apart, a maximum plasma concentration in steady state (Cmax) of Acetylsalicylic acid of 328.25±5.69 ng/mL after median tₘₐₓ of 0.83 h and an area under the plasma concentration-time curve from time zero to infinity in steady state (AUC) of 531.75±2.80 ng·h/mL, as determined by human PK studies.
(40) The pharmaceutical composition according to any one of (1) to (39), wherein the pharmaceutical composition has an in vitro dissolution rate of rivaroxaban or Acetylsalicylic acid of 85 %, based on the total rivaroxaban or Acetylsalicylic acid content, respectively, of the single dosage form, as determined by using European Pharmacopoeia edition 10 Apparatus Baskets "1" in 900 mL volume of Acetate buffer pH 4.5, 37°C ± 0.5°C, 100 RPM or Apparatus type: 2 paddles with sinkers in 900 mL volume of Acetate buffer pH 4.5, 37°C± 0.5°C, 75 RPM
(41) According to a further aspect, the present invention relates to a pharmaceutical composition as defined in any one of (1) to (40) for use in a prevention of atherothrombotic events in adult patients after an acute coronary syndrome (ACS) with elevated cardiac biomarkers and/or for the prevention of atherothrombotic events in adult patients with coronary artery disease (CAD) or symptomatic peripheral artery disease (PAD) at high risk of ischaemic events
(42) The pharmaceutical composition for use according to (41), wherein the pharmaceutical composition is administered orally twice daily preferably 11 to 13 hours apart, and more preferably 12 hours apart.

The invention also relates to a combined use of 2.5 mg of rivaroxaban and 50 mg of acetylsalicylic acid in CAD, PAD therapy or in a therapy of reducing the risk of myocardial infarction, stroke or cardiovascular death in a human patient with coronary artery disease and/or peripheral artery disease including patients after ACS. By such a combined use it is understood that 2.5 mg of rivaroxaban and 50 mg of acetylsalicylic acid are administered orally together in form of two separate dosage forms taken together or subsequently one after the other, within a short period of time such as up to 30 seconds or 1 minute or up to 5 minutes which is reasonable to perform oral administration of two dosage forms. It is however preferred that the separate dosages of respectively 2.5 mg of rivaroxaban and 50 mg of acetylsalicylic acid are taken orally together at the same time and swallowed at the same time.

It is even more preferred that 2.5 mg of rivaroxaban and 50 mg of acetylsalicylic acid are in a form of one fixed-dose pharmaceutical composition.

Thus, the invention relates also to the:
(43) Combination of 2.5 mg of rivaroxaban and 50 mg of acetylsalicylic acid for use in a therapy of reducing the risk of myocardial infarction, stroke or cardiovascular death in a human patient with coronary artery disease and/or peripheral artery disease, comprising the dosage regimen of administering orally to the human patient twice daily, preferably 11 to 13 hours apart.
(44) The combination for use according (43), wherein the dosage regimen of administering is 12 hours apart.
(45) The combination for use according to (43) or (44), wherein 2.5 mg of rivaroxaban and 50 mg of acetylsalicylic acid are in a form of a fixed-dose pharmaceutical composition.

According to a preferred aspect of the present invention, the pharmaceutical composition for oral administration has the features according to the above (1) and (2). Thus, a the fixed-dose pharmaceutical composition for oral administration is provided, which comprises: 2.5 mg of rivaroxaban and 50 mg of acetylsalicylic acid and the composition is in form of a tablet. The pharmaceutical composition for oral administration according to this aspect may be further characterized according to any one of the above (3) to (7) and (10) to (40). According to a further aspect, the said pharmaceutical composition is for use in a method of treatment and prevention of atherothrombotic events. The pharmaceutical composition for use may be further characterized according to any one of the above (41) to (42).

According to a further preferred aspect of the present invention, the pharmaceutical composition for oral administration has the features according to the above (1), (2) and (5). Thus, a the fixed-dose pharmaceutical composition for oral administration is provided, which comprises: 2.5 mg of rivaroxaban and 50 mg of acetylsalicylic acid and the composition is in form of a tablet, which is coated with and active coating comprising rivaroxaban. The pharmaceutical composition for oral administration according to this aspect may be further characterized according to any one of the above (3) to (7) and (10) to (40). According to a further aspect, the said pharmaceutical composition is for use in a method of treatment and prevention of atherothrombotic events. The pharmaceutical composition for use may be further characterized according to any one of the above (41) to (42). According to an another preferred aspect of the present invention, the pharmaceutical composition for oral administration has the features according to the above (1) and (8). Thus, a the fixed-dose pharmaceutical composition for oral administration is provided, which comprises: 2.5 mg of rivaroxaban and 50 mg of acetylsalicylic acid and the composition is in form of a capsule. The pharmaceutical composition for oral administration according to this aspect may be further characterized according to any one of the above (9) and (10) to (40). According to a further aspect, the said pharmaceutical composition is for use in a method of treatment and prevention of atherothrombotic events. The pharmaceutical composition for use may be further characterized according to any one of the above (41) to (42).

According to a yet another preferred aspect of the present invention, the pharmaceutical composition for oral administration has the features according to the above (1), (8) and (9). Thus, a the fixed-dose pharmaceutical composition for oral administration is provided, which comprises: 2.5 mg of rivaroxaban and 50 mg of acetylsalicylic acid and the composition is in form of a capsule, which contains two tablets of respectively component a) and component b). The pharmaceutical composition for oral administration according to this aspect may be further characterized according to any one of the above (9) and (10) to (40). According to a further aspect, the said pharmaceutical composition is for use in a method of treatment and prevention of atherothrombotic events. The pharmaceutical composition for use may be further characterized according to any one of the above (41) to (42). According to a preferred aspect of the present invention a combination for use in a method of treatment and prevention of atherothrombotic events has the feature according to the above (43) and (44). Thus, a combination of 2.5 mg of rivaroxaban and 50 mg of acetylsalicylic acid for use in a therapy of reducing the risk of myocardial infarction, stroke or cardiovascular death in a human patient with coronary artery disease and/or peripheral artery disease is provided, which is to be administered orally to the human patient twice daily, preferably 11 to 13 hours apart.

According to another preferred aspect of the present invention a combination for use in a method of treatment and prevention of atherothrombotic events has the feature according to the above (43), (44) and (45). Thus, a fixed dose combination of 2.5 mg of rivaroxaban and 50 mg of acetylsalicylic acid for use in a therapy of reducing the risk of myocardial infarction, stroke or cardiovascular death in a human patient with coronary artery disease and/or peripheral artery disease is provided, which is to be administered orally to the human patient twice daily, preferably 11 to 13 hours apart combination.

### Detailed description

According to an aspect of the present invention, a fixed-dose pharmaceutical composition for oral administration is provided, the composition comprising 2.5 mg of rivaroxaban and 50 mg of Acetylsalicylic acid, and pharmaceutically acceptable excipients.

An immediate release (IR) component releases active substances with no special rate controlling features. The understanding of the terms corresponds to common pharmaceutical handbook definitions. The immediate release component shows a release of the active substance, which is not deliberately modified by a special formulation and/or manufacturing method. The immediate-release (IR) component ensures that rivaroxaban and/or Acetylsalicylic acid are available to the body without relevant impact of the dosage form. In particular, according to the present invention, the IR component achieves in vitro dissolution of >85% wt.% of the active within 15 or 30 minutes, based on the total content of the active ingredient in the respective component a) or b). Specifically, dissolution of Rivaroxaban after 30 minutes is ≥ 85% (S1= Q + 5; Q = 80%) and dissolution of Acetylsalicylic acid after 15 minutes is ≥ 85% (S1= Q + 5; Q = 80%).

The aforementioned in vitro dissolution rates are determined by using European Pharmacopoeia edition 10 Apparatus Baskets "1" in 900 mL volume of Acetate buffer pH 4.5, 37°C ± 0.5°C, 100 RPM or Apparatus type: 2 paddles with sinkers in 900 mL volume of Acetate buffer pH 4.5, 37°C ± 0.5°C, 75 RPM.

The combination of 2.5 mf of rivaroxaban in form of immediate release component a) and 50 mg of Acetylsalicylic acid in form of immediate release component according to the present invention provides a fixed dose pharmaceutical composition. The fixed-dose form shows, after oral administration, an immediate release of both active ingredients at the same time compared to that of prior art immediate release dosage forms of both active ingredients administered by the same route but administered in different dosage ratios and using different timing regimen. In particular, the pharmaceutical composition according to the invention achieves in vitro dissolution rates of Rivaroxaban after 30 minutes is ≥ 85% (S1= Q + 5; Q = 80%) and dissolution of Acetylsalicylic acid after 15 minutes is ≥ 85% (S1= Q + 5; Q = 80%) based on the total content of the active ingredient in the respective component a) or b). The in vitro dissolution rates are determined by using European Pharmacopoeia edition 10 Apparatus Baskets "1" in 900 mL volume of Acetate buffer pH 4.5, 37°C ± 0.5°C, 100 RPM or Apparatus type: 2 paddles with sinkers in 900 mL volume of Acetate buffer pH 4.5, 37°C ± 0.5°C, 75 RPM.

The inventors have found that a more uniform release of both active substances can be achieved by the use of fixed combination of IR 2.5 mg of rivaroxaban component and IR 50 mg component of Acetylsalicylic acid. The fixed dose combination according to the invention, when administered twice daily, provides shorter dosing interval between the subsequent dosages of Acetylsalicylic acid and thus, consequently, smaller difference in platelet aggregation in the twice-daily regimen compared to the once-daily administration of Acetylsalicylic acid. The administration of specifically 50 mg of Acetylsalicylic acid being the half of the clinically tested daily dose which should be taken as daily dose in CAD/PAD therapy and after ACS, ensures uniform levels in serum of Acetylsalicylic acid over the day and this level is ensured exactly at the time when rivaroxaban starts to be available in serum

Additionally, the administration of 2.5 mg of rivaroxaban and 50 mg of Acetylsalicylic acid as a one dosage form ensures better patients' compliance which is of utmost importance in anticoagulant and antithrombotic therapies.

Thus, fixed dose combination (FDC) of rivaroxaban and Acetylsalicylic acid (2.5 mg + 50 mg) can serve as a substitution therapy in coronary artery disease (CAD), symptomatic peripheral artery disease (PAD) and patients after an acute coronary syndrome (ACS) with the benefits of minimizing the risk of inappropriate administration of the second component of the therapy and provision of a more consistent platelet inhibition by Acetylsalicylic acid compared with standard once-daily dosing in patients recommended in the prior art.

The pharmacokinetics of the pharmaceutical composition and dosage regimen of the present invention thus differ fundamentally from the pharmacokinetics of prior art pharmacokinetics achieved by dosing 2.5 mg of rivaroxaban with 75-100 mg of Acetylsalicylic acid.

The inventors have surprisingly found that, due to the use of the fixe-dose composition according to the invention, the dosage frequency can be reduced to 2 times daily (BID) with the use of only one dosage form, whereas prior art dosage for combined rivaroxaban and Acetylsalicylic acid therapy require administration of rivaroxaban twice daily and additionally Acetylsalicylic acid once daily.

According to an embodiment, the pharmaceutical composition is a single dosage form
in the form of tablet. In the tablet, the component a) forms a first layer, the component b) forms a second layer, and the first layer at least partially covers the surface of the second layer. The second layer may be completely surrounded by the first. However, it is preferable that the tablet is a bilayer tablet, which means that the first layer is arranged on one side (i.e., one of the two faces) of the second layer and, typically, covers this side completely. Such a tablet can be produced economically.

The tablet may be uncoated or coated. The coating may, for example, be a film-coating. The tablet may have any suitable coating, e.g., functional coating. Such coatings can be found in the "Pharmaceutical Manufacturing Handbook" edited by Shayne Cox Gad, published by John Wiley & Sons, Inc., Hoboken, New Jersey, March 2008.

Preferably, the tablet is uncoated. In the pharmaceutical composition, the component a) and the component b) may be compressed together, for example, by using a bilayer-tableting machine. The component a) and the component) may also be granulated and then be compressed to a tablet, for example, by using a bilayer-tableting machine.

Preferably, the component a) is contained as granulates, and the component b) is contained as granulates, in the pharmaceutical composition, in particular, when the pharmaceutical composition is a bilayer tablet as the single dosage form. The granulates may be obtained by common dry or wet granulation techniques, preferably by wet granulation. In addition to granulates, the component a) and/or the component b) may also contain an extragranular fraction, for example, comprising one or more of a lubricant, a filler, an absorbent and a glidant. The components of an extragranular fraction are added after formation of the granulates. The layers of the tablet may be obtained as well be a well know direct compression method.

The pharmaceutical composition according to this embodiment is particularly suitable for a dosage regimen, where a single dosage form is administered two times daily, preferably, 11 o 13 hours, most preferably 12 hours apart. It allows for particularly constant, therapeutic plasma and blood concentrations of both active ingredients, which improves the efficacy of the treatment of CAD and PAD, also in patients after ACS, and reduces the consequences of lack of compliance to the dosing regimen, namely consequences of a dose being skipped or being taken excessively due to the memory shortages or by omission.

In the pharmaceutical composition, the component a) and the component b) each comprise at least one excipient. As the at least one excipient, the component a) may comprise at least one of a filler, a disintegrant, a binder, a lubricant, a glidant and any combination thereof. Preferably, the component a) comprises a filler, a disintegrant, a binder, a lubricant, and, optionally, a glidant.

The component a) preferably consists of rivaroxaban and a filler, a disintegrant, a binder, a lubricant, and, optionally, a glidant.

The component b) may comprise, as the at least one excipient, at least one of a filler, a disintegrant, a binder, a lubricant, a glidant and any combination thereof, preferably the component b) comprises a filler, a disintegrant, a, a glidant lubricant, and, optionally, a binder. The component b) preferably consists of Acetyl salicylic acid, , a filler, a disintegrant, a glidant, a lubricant, and, optionally, a binder. More preferably, the component b) does not contain a binder.

In the pharmaceutical composition, the component a) and/or, preferably and, the component b) comprise a filler. Herein, a filler means at least one filler. Accordingly, the filler may be at least one filler independently selected from the group consisting of anhydrous or monohydrate lactose, sucrose, microcrystalline cellulose, starch such as maize starch, pregelatinized starch, microcrystalline cellulose coated with colloidal silica, mannitol, sorbitol and any combination thereof. Preferably, the filler is at least one independently selected from the group consisting of anhydrous or monohydrate lactose, mannitol, microcrystalline cellulose, maize starch, saccharose, sorbitol and any combination thereof.

More preferably, the filler is at least one selected from the group consisting of lactose monohydrate, microcrystalline cellulose, or a combination thereof. The component a) may contain the filler in an amount of 10 to 50 wt.%, preferably 10 to 30 wt.%, based on the weight of the component a).

The component b) contains the filler in an amount of 10 to 45 wt.%, preferably 10 to 30 wt.%, based on the weight of the component b).

In the pharmaceutical composition, the component a) and/or the component b) comprise a binder. Preferably the component a) comprises a binder and the component b) is free of a binder. Accordingly, the binder may, for example, be at least one binder independently selected from the group consisting of hydroxypropylcellulose, hydroxypropylmethylcellulose (Hypromellose), methylcellulose, carboxymethylcellulose, hydroxycellulose, hydroxyethylcellulose, polyvinyl alcohol, polyvinylpyrrolidone, copovidone, pregelatinized starch, gelatine and any combination thereof. Preferably, the binder is at least one independently selected from the group consisting of hydroxypropyl cellulose, polyvinylpyrrolidone and any combination thereof. More preferably the binder is Hypromellose. The binder may be used in an amount equal to 0.4 to 10 wt.%, preferably 0.8 to 8 wt.%, more preferably 0.8 or 0.9 or 0.5 wt.%, based on the total mass of the composition. The component a) may contain the binder in an amount of 0.4 to 5 wt.%, preferably 0.4 to 3 wt.%, more preferably 0.5 wt.%, based on the weight of the component a). The component b) may contain the binder in an amount of 0 of 0.4 to 10 wt. % preferably 0.8 to 8 wt.%, also preferably 0.7 or 0.9 or 0.5 wt.% based on the weight of the component a). A binder is not required if the respective component is produced by dry granulation or direct compacting. It is however preferred to use wet granulation in the preparation of the components, where a binder is commonly used as described in more detail below.

In the pharmaceutical composition, the component a) and/or, preferably and, the component b) comprise a disintegrant. Herein, a disintegrant means at least one disintegrant. Accordingly, the disintegrant may, for example, be at least one disintegrant independently selected from the group consisting of carboxymethylcellulose sodium salt, sodium croscarmellose, microcrystalline cellulose, crospovidone, sodium starch glycolate, maize starch, and any combination thereof. Preferably, the disintegrant is at least one independently selected from the group consisting of sodium starch glycolate, sodium croscarmellose, maize starch and any combination thereof. More preferably, the disintegrant is sodium croscarmellose or sodium starch glycolate. The disintegrant may be used in an amount of 0.5 to 25 wt.%, preferably 2 to 16 wt.%, more preferably 5 wt.%, based on the total mass of the composition. The component a) may contain the disintegrant in an amount of 0.5 to 6 wt.%, preferably 2 to 6 wt.%, more preferably 5 wt.%, based on the weight of the component a). The component b) may contain the disintegrant in an amount of 0.3 to 5 wt.%, preferably 2 to 5 wt.%, more preferably 3 wt.%, based on the weight of the component b).

In the pharmaceutical composition, the component a) and/or, preferably and, the component b) comprise a lubricant. Herein, a lubricant means at least one lubricant. Accordingly, the lubricant may, for example, be at least one lubricant independently selected from the group consisting of stearic acid, magnesium stearate, calcium stearate, sodium stearyl fumarate, glycerol tristearate, palm oil derivatives such as palmitic acid or hydrogenated palm oil, especially Softisan 154, hydrogenated vegetable oil, such as hydrogenated castor oil, and any combination thereof. Preferably, the lubricant is at least one independently selected from magnesium stearate and stearic acid. The lubricant may be used in an amount of 0.1 to 10 wt.%, preferably 0.1 to 5 wt.%, more preferably 0.5, 1.3 to 1.4 wt.% such as 1.35 wt.%, based on the total mass of the composition. The component a) may contain the lubricant in an amount of 0.1 to 5 wt.%, preferably 2.3; 0.45; 0.5 wt.%, based on the weight of the component a).

The component b) may contain the lubricant in an of 0.1 to 5 wt.%, preferably 0.45 wt.%, based on the weight of the component b).

In the pharmaceutical composition, the component a) and/or the component b) may comprise a glidant. It is preferred that the component b) but not the component a) comprises a glidant. Herein, a glidant means at least one glidant. Accordingly, the glidant may, for example, be at least one glidant independently selected from the group consisting of fumed silica (colloidal silicon dioxide), such as colloidal anhydrous silica, starch, talc and any combination thereof.

Preferably, the glidant is colloidal anhydrous silica. The glidant may be used in an amount of 0.1 5 wt.%, preferably 0.4 to 2 wt.%, more preferably 0.46 wt.%, based on the total mass of the composition. The component a) may contain the glidant in an amount of 0.1 to 5.0 wt.%, preferably 1.6 or 1.0 or 0.9 wt. % based on the weight of the component a). The component b) may contain the glidant in an amount of up to 0.9 or 1.3 wt.%, based on the weight of the component b).

Particularly preferred pharmaceutical compositions of the present invention are obtained by combining preferred embodiments such as preferred contents and preferred components. For example, according to a particularly preferred embodiment, the pharmaceutical composition is a single dosage form in the form of a bilayer tablet, in which the component a) forms a first layer, the component b) forms a second layer.

According to particularly preferred embodiment, the component a) component a) has a rivaroxaban content of 1 to 5 wt.%, preferably 1.5 to 4.5 wt.%, more preferably 2.6 to 3.1 wt.%, such as 3 wt.%, based on the total weight of the component a); furthermore, the component b) has an acetylsalicylic acid content of 20 to 80 wt.%, preferably 35 to 75 wt.%, more preferably 33 to 72 wt.%, such as 55.6 wt.%, based on the total weight of the component b).

Also preferably, the component a) has a rivaroxaban content of 0.2 to 5 wt.%, preferably 0.35 to 2.5 wt.%, more preferably 0.5 to 1.5 wt.%, such as 0.55 wt.%, based on the total weight of the film-coated tablet furthermore the component b) has an acetylsalicylic acid content of 5 to 25 wt.%, preferably 10 to 20 wt.%, more preferably 11 to 15 wt.%, such as 11.2 wt.%, based on the total weight of the film-coated tablet

According to another particularly preferred embodiment, the component a) has a rivaroxaban content of 0.5 to 5 wt.%, preferably 0.8 to 2.5 wt.%, more preferably 1 to 1.5 wt.%, such as 1.1 wt.%, based on the total weight of the bilayer tablet furthermore the component b) has an acetylsalicylic acid content of 10 to 50 wt.%, preferably 15 to 35 wt.%, more preferably 20 to 25 wt.%, such as 22.7 wt.%, based on the total weight of the bilayer tablet.

Preferably, the pharmaceutical composition of the present invention has an in vitro dissolution rate of rivaroxaban or Acetylsalicylic acid of 85 %, based on the total rivaroxaban or Acetylsalicylic acid content, respectively, of the single dosage form, as determined by using European Pharmacopoeia edition 10 Apparatus Baskets "1" in 900 mL volume of Acetate buffer pH 4.5, 37°C ± 0.5°C, 100 RPM or Apparatus type: 2 paddles with sinkers in 900 mL volume of Acetate buffer pH 4.5, 37°C ± 0.5°C, 75 RPM

In another aspect, the invention relates to the method of manufacturing the pharmaceutical composition. The method enables the production of the pharmaceutical composition using conventional tools and well-known pharmaceutical technologies, while significantly simplifying the manufacturing process, and reducing its costs comparing to manufacturing standards known in the art. The pharmaceutical composition, in particular in the form of a bilayer tablet, can be prepared by a method comprising direct compression, dry granulation or wet granulation for making the immediate-release component and the component b).

Thus, a method of manufacturing the prolonged-release pharmaceutical composition comprises (a) a step of preparing the component a); (b) a step of preparing the component b); (c) a step of combining the component a) and the component (b) to form a bilayer tablet, in which the component a) forms a first layer, the component (b) forms a second layer; and, optionally, (d) a step of forming a coating around the first and second layers obtained in step (c). Preferably, no coating is formed, that is, step (d) is preferably not carried out.

In step (a) and step (b), the component a) and component b) may be independently prepared using a dry or wet technology such as dry or wet granulation.

A dry-granulated component a) or component b) can be obtained using direct dry granulation of a powder mixture comprising active ingredient or a pharmaceutically acceptable salt thereof, suitable excipients as described above. Other excipients can be also included, if needed. Suitable excipients, used in pharmacy, can be found in the "Handbook of Pharmaceutical Excipients" edited by R. C. Rowe, P. J. Sheskey, and S. Owen, Pharmaceutical Press, edition VII. The dry granulation method used to obtain the component (step (a)) or the component (step (b)) can be selected from roller compaction and slugging. Methods known to the skilled person and are described, for example, in the "Pharmaceutical Manufacturing Handbook" edited by Shayne Cox Gad, published by John Wiley & Sons, Inc., Hoboken, New Jersey.

A wet-granulated component a) or component b) can be obtained using a suitable granulation method known in the art using powder mixtures comprising rivaroxaban or acetylsalicylic acid, suitable excipients as described above.

Other excipients can be also included, if needed. The wet granulation method used to obtain the component a) (step (a)) or the component b) (step (b)) can be selected from low shear, high shear, and fluid bed granulation. Preferably, the component a) and the component b) are obtained by wet granulation (steps (a) and (b)).

Blending operations can be performed in a suitable blender, preferably in a container
blender.

According to an embodiment of the method, the component a) and/or the component b) are prepared using dry granulation (steps (a) and (b)). In such a variant, the method preferably comprises the following steps: (I) blending Acetylsalicylic acid or a pharmaceutically acceptable salt thereof with all excipients except lubricant and, to homogeneity; II) adding a part of the lubricant and subsequent blending; III) dry granulation by roller-compaction or slugging; IV) breaking down the resulting slugs or sheets using a milling technique to produce granules; V) adding the remaining part of lubricant and subsequent blending.

In the dry methods of granulation, the primary powder blend particles are aggregated under high pressure using one of two main dry granulation processes: either roller-compaction or slugging. In both cases the intermediate products, slugs or sheets, are broken down using a suitable milling technique to produce granular material, which can be additionally sieved to separate the desired size fraction. Such a way of processing the active ingredients powder blend is very advantageous for several reasons. It allows for the elimination of water, which may affect active ingredients stability, and obtaining a granulate having grain sizes that are optimal for further processing (combination with the second component and optional extragranular phase component without the effect of physical segregation). Additionally, a broad ratio (wt.%) of active ingredient can be blended into the dry granulate intermediate, which is not possible in other dry pharmaceutical processes (e.g. direct compression).

According to another embodiment of the method, the component a) and/or the component b) are prepared using wet granulation (steps (a) and (b)). In such a variant, the method preferably comprises the following steps: I) mixing of active ingredient or a pharmaceutically acceptable salt thereof with at least one filler, at least one disintegrant, optionally further excipients, in a high shear granulator to homogeneity; II) granulating the mixture of step I) by addition of a binder solution in water or another processing agent, such as an alcohol, e.g. ethanol, or with a mixture thereof; III) drying and unifying the dried granule sizes by sieving or screening; and, optionally, IV) adding an extragranular phase comprising lubricant and/or a mixture of filler and glidant and/or an absorbent and a glidant. Active ingredient may be also subjected to a process of wet granulation (e.g., low shear, high shear or in a fluidized bed), to improve its solubility. Wet granulation facilitates obtaining the desired in vitro and in vivo release profiles. The resulting wet granulate is dried and, for example, screened to obtain a proper distribution of the particles, allowing a durable combination with the other component and additional excipients.

According to a preferred embodiment of the method, the component a) is prepared
using wet granulation (step (a)), and the component b) is prepared using dry granulation (step (b)). It is also preferred to prepare the component a) (step (a) and the component b)
(step (b)) by wet granulation.

In step (c) of the method, the resulting components a) and b) are combined together, and
depending on the desired final form, for example, compressed into separate layers in a tablet, or separate tablets or pellets to be packed together into a hard capsule; optionally followed by forming a coating in step (d), resulting in a single unit dosage form.

Preferably, the fixed-dose pharmaceutical composition is prepared by using a bilayer tableting technology in step (c) or by simple tabletting followed by packing into hard capsules.

The inventors have found that a more uniform release of both active substances can be achieved by the use of fixed combination of IR 2.5 mg of rivaroxaban component and IR 50 mg component of Acetylsalicylic acid.

Additionally, the administration of 2.5 mg of rivaroxaban and 50 mg of Acetylsalicylic acid as a one dosage form ensures better patients' compliance which is of utmost importance in anticoagulant and antithrombotic therapies.

According to another aspect, the present invention provides a method of reducing the risk of myocardial infarction, stroke or cardiovascular death in a human patient with coronary artery disease and/or peripheral artery disease using a pharmaceutical composition as described herein above. Thus, the present invention provides a pharmaceutical composition as described herein above for use in a method of prevention of atherothrombotic events in adult patients after an acute coronary syndrome (ACS) with elevated cardiac biomarkers or for the prevention of atherothrombotic events in adult patients with coronary artery disease (CAD) or symptomatic peripheral artery disease (PAD).

According to an embodiment, the pharmaceutical composition is a single dosage form, such as a bilayer tablet or a capsule comprising 2 tablets or pellets of the respective active ingredients, which is administered orally two times daily, preferably 11 to 13 hours apart, more preferably 12 hours apart. Typically, the pharmaceutical composition is administered for 1 day, preferably for 5 consecutive days and the therapy is continued up to 1 year, 2 years or 3 years or longer. This regimen has the advantage of better patients' compliance and improved quality of life during therapy in comparison to the prevention of atherothrombotic events in adult patients after an acute coronary syndrome (ACS) with elevated cardiac biomarkers or for the prevention of atherothrombotic events in adult patients with coronary artery disease (CAD) or symptomatic peripheral artery disease (PAD) using a prior art active ingredient composition.

In conventional therapies, the high dosages of rivaroxaban and acetylsalicylic acid requires usage of two separate dosage forms, with the necessity of taking 3 tablets during a day, instead of 2.

Typically, the pharmaceutical composition is administered with or without food. In combination with food means that the pharmaceutical composition is taken shortly before, together with or after the meal. It is thus preferred that the pharmaceutical composition is a single dosage form, which is administered orally within an interval of from 30 min or longer before a meal and 30 min or longer after a meal. Preferably, the pharmaceutical composition is administered between at a meal and 30 min after a meal, and more preferably, right after the meal, that is, within 5 min after a meal or irrespective of the meal.

The method of treatment according to the present invention thus provides favourable Rivaroxaban and acetylsalicylic acid pharmacokinetics, which are not obtained by prior art dosage regimens and dosages of the both actives.

Preferably, wherein oral administration of one single dosage form of the pharmaceutical
composition provides after oral administration, a maximum plasma concentration of rivaroxaban (Cmax) of 77.69±28.2 ng/mL after median tmax of 2 hours after administration, and an area under the plasma concentration time curve from time zero to infinity of 481.26±22.3 ng·h/mL, as determined by human PK studies described below. It is further preferred that the pharmaceutical composition according to any one of (1) to (38), wherein the pharmaceutical composition provides, after oral administration of a single dosage form of the pharmaceutical composition twice a day and preferably 12 hours apart, a maximum plasma concentration in steady state (Cmax) of
Acetylsalicylic acid of 328.25±5.69 ng/mL after median tmax of 0.83 h and an area under the plasma concentration-time curve from time zero to infinity in steady state (AUC) of 531.75±2.80 ng·h/mL, as determined by human PK studies described below.

### I. TABLETS IN CAPSULE

Drug product in form of capsule with acetylsalicylic acid in strength 50 mg and rivaroxaban in strength 2.5 mg, Drug product formulation components:

| **Drug product form with acetylsalicylic acid in strength 50 mg** | | | |
|---|---|---|---|
| **Component b) of ASA 50 mg** | **Function** | **Alternative type of the component** | **Different components** |
| Acetylsalicylic acid | active substance | - | - |
| Cellulose microcrystalline (type 102) (mean particle size: 100 µm) | filler | Cellulose (mean particle size: 20- 250 µm) | powdered cellulose, silicified microcrystalline cellulose |
| Starch, maize (native) (mean particle size: 10-15 µm) | filler, disintegrant | Starch (mean particle size: 5- 45 µm) | potato starch, rice starch, pea starch, tapioca starch |
| Silica, colloidal anhydrous (SSA 175 - 225 m²/g) | glidant | Hydrophilic fumed Silica G (SSA 75 - 410 m²/g) | hydrophobic fumed Silica G, fumed mixed oxides |
| Stearic acid (50) (stearic acid content: 40 - 60%) | lubricant | Stearic acid (stearic acid content: 40-100%) | palmitic acid, lauric acid, myristic acid |

| **Drug product form with rivaroxaban in strength 2.5 mg** | | | |
|---|---|---|---|
| **Component a) of RIVA 2.5 mg** | **Function** | **Alternative type of com ponents** | **Different components** |
| Rivaroxaban | active substance | - | - |
| Lactose monohydrate (mean particle size: about 40 µm) eventually (bulk density: 0.6 g/ml) | filler | Lactose mean particle size: 15- 140 µm) eventually bulk density: 0.4-0.8 g/ml) | lactose anhydrous, lactose spray-dried |
| Cellulose microcrystalline (type 101) mean particle size: 50 - 70 µm | filler | Cellulose (mean particle size: 20 - 250 µm) | powdered cellulose, silicified microcrystalline cellulose |
| Croscarmellose sodium (degree of substitution: 0.79) | disintegrant | Croscarmellose sodium (degree of substitution: 0.60 - 0.85) | carboxymethylcellulose sodium, carboxymethylcellulose calcium |
| Hypromellose (E 5) (viscosity: 4-6 mPa s) | binder | Hypromellose (viscosity: 3-140 000 mPa s) | hydroxypropyl cellulose, hydroxyethyl cellulose, hydroxyethyl methyl cellulose |
| Sodium Lauryl sulphate (Texapon K-12) | solubilizer | | magnesium lauryl sulphate, cetyl alcohol, cetostearyl alcohol |
| Magnesium Stearate (specific surface area: 4-10 m²/g) | lubricant | Magnesium Stearate (specific surface area: 1 - 15 m²/g) | calcium stearate, zinc stearate, stearic acid, magnesium aluminium Silica |
| Purified Water ¹ | processing agent | | carbon dioxide free water, deaerated water, hard water, soft water, water for injection |

| | | | |
|---|---|---|---|
| ¹ Purified water is processing agent using in granulation process and essentially removed during the manufacturing process. | | | |

### Drug product form (TABLETS IN CAPSULE):

1. Both tablets are coated: ASA, film-coated tablet & RIVA, film-coated tablet
2. One tablet is coated:
   a. ASA, tablet & RIVA, film-coated tablet
   b. ASA, film-coated tablet & RIVA, tablet

### CAPSULES QUALITY

1. Gelatine Capsules Hard
2. HPMC Capsules Hard

| **Component b) Acetylsalicylic acid, 50 mg**, **tablet IR** (immediate release) | | |
|---|---|---|
| **Component** | **mg/tab.** | **[%]** |
| Acetylsalicylic acid | 50.000 | 35 - 70 |
| Cellulose microcrystalline | - | 10 - 90 |
| Starch, maize | - | 5-75 |
| Silica G, colloidal anhydrous | - | 0.1 - 5 |
| Stearic acid | - | 0.1 - 5 |
| Film-coated layer | - | 0-5 |
| **Total mass** | **70** - **150.0** | **100** |

| **Component a) Rivaroxaban, 2.5 mg, tablet IR** | | |
|---|---|---|
| **Component** | **mg/tab.** | **[%]** |
| Rivaroxaban | 2.5 | 1.5 - 3.5 |
| Lactose monohydrate | - | 5-90 |
| Cellulose microcrystalline | - | 5-90 |
| Croscarmellose sodium | - | 0.5 - 25 |
| Hypromellose | - | 0.5 - 10 |
| Sodium Lauryl sulphate | - | 0.5 - 10 |
| Magnesium Stearate | - | 0.1 - 5 |
| Film-coated layer | - | 0-5 |
| **Total mass** | **80** - **160.0** | **100** |

### Sample composition:

| **Component b) Acetylsalicylic acid, 50 mg, tablet IR** (immediate release) | | |
|---|---|---|
| **Component** | **mg/tab.** | **[%]** |
| Acetylsalicylic acid | 50.0 | 55.6 |
| Cellulose microcrystalline | 25.5 | 28.3 |
| Starch, maize | 13.0 | 14.4 |
| Silica G, colloidal anhydrous | 1.0 | 1.1 |
| Stearic acid | 0.5 | 0.6 |

| **Component a) Rivaroxaban, 2.5 mg, tablet IR** | | |
|---|---|---|
| **Component** | **mg/tab.** | **[%]** |
| Rivaroxaban | 2.5 | 3 |
| Lactose monohydrate | 25.0 | 30 |
| Cellulose microcrystalline | 44.5 | 53.5 |
| Croscarmellose sodium | 4.0 | 5 |
| Hypromellose | 1.6 | 2 |
| Sodium Lauryl sulphate | 1.6 | 2 |
| Magnesium Stearate | 0.8 | 1 |
| Film-coated layer | 3.0 | 3.5 |

### MANUFACTURING

### 1. Tablets with ASA (immediate release) - Direct Compression Technology

Drug product excipients (except of lubricant) are sieving and mixing. Active substance is sieving. The drug product components (API + excipients except of lubricant) are placed in suitable container and blend until homogenous mixture is obtained. Lubricant is sieved into the mixture and blend to obtain tableting mass. The tableting mass is compressed using a tablet press to obtain tablets with the desired strength. The tablets with ASA may be film-coated.

### 2. Tablets with RIVA (immediate release) - Wet Granulation Technology

The specified amounts of active substance (rivaroxaban) and intra-granular excipients are mixed with the aid of high-shear granulator to obtain the powder blend. The binder solution is sprayed on the powder bland during mixing in high-shear granulator. Then the main granulation phase is performed. The wet mass is transferred into the fluid bed dryer and the drying process is performed. The dried mass is passed through a screen into a suitable container. Lubricant is sieved into the mixture and blend to obtain tableting mass. The tableting mass is compressed using a tablet press to obtain tablets with the desired strength. The tablets with RIVA may be film-coated.

### 3. Encapsulation

Tablet or tablets (or film-coated ones) with ASA as well as with RIVA are putted into capsule to obtain the drug product with desired strengths of active substances. The process is performed with the aid of encapsulator.

### II. FILM-COATED TABLET (MULTI_COATING)

Drug product in form of film-coated tablet with acetylsalicylic acid in strength 50 mg (tablet core) and rivaroxaban in strength 2.5 mg (API layer)

Drug product formulation components:

| **Drug product form with acetylsalicylic acid in strength 50 mg (tablet core)** | | | |
|---|---|---|---|
| **Component b) of ASA 50 mg** | **Function** | **Alternative type of the com ponent** | **Different components** |
| Acetylsalicylic acid | active substance | - | - |
| Cellulose microcrystalline (type 102) (mean particle size: 100 µm) | filler | Cellulose (mean particle size: 20 - 250 µm) | powdered cellulose, silicified microcrystalline cellulose |
| Starch, maize (native) (mean particle size: 10 - 15 µm) | filler, disintegrant | Starch (mean particle size: 5-45 µm) | potato starch, rice starch, pea starch, tapioca starch |
| Silica G, colloidal anhydrous (SSA 175 - 225 m²/g) | glidant | Hydrophilic fumed Silica G (SSA 75 - 410 m²/g) | hydrophobic fumed Silica G, fumed mixed oxides |
| Stearic acid (50) (stearic acid content: 40 - 60%) | lubricant | Stearic acid (stearic acid content: 40 - 100%) | palmitic acid, lauric acid, myristic acid |

| **Drug product form with rivaroxaban in strength 2.5 mg** | | | |
|---|---|---|---|
| **Component a) of RIVA 2.5 mg** | **Function** | **Alternative type of com ponents** | **Different components** |
| Rivaroxaban | active substance | - | - |
| Film-coating system | coating | Based on polyvinyl alcohol (PVA) or Hypromellose, (HPMC) or hydroxypropyl cellulose (HPC) or mixture thereof | Polyvinyl alcohol, Methylcellulose Hypromellose, Hydroxypropyl cellulose, Sodium Carmellose, Macrogol, Polyvinylpyrrolidone, Dimethylaminomethacrylate, Methacrylic acid polyesters, Ethylcellulose, Cellulose acetate |
| Purified Water ¹ | processing agent | water, | carbon dioxide free water, deaerated water, hard water, soft water, water for injection, water for irrigation |

| | | | |
|---|---|---|---|
| ¹ Purified water is processing agent using in granulation process and essentially removed during the manufacturing process. | | | |

### Drug product form (FILM-COATED TABLET):

- ASA 50 mg IR tablet core
- Sub-coating layer
- RIVA 2.5 mg IR layer
- Top-coating layer

| **Component b) Acetylsalicylic acid, 50 mg, tablet core IR** (immediate release) | | | |
|---|---|---|---|
| **Component** | **mg/tab.** | **[%]** | **Function** |
| Acetylsalicylic acid | 50.000 | 10-25 | active substance |
| Cellulose microcrystalline | - | 10-90 | filler |
| Starch, maize | - | 3-75 | filler, disintegrant |
| Silica G, colloidal anhydrous | - | 0.1 - 1 | glidant |
| Stearic acid | - | 0.1 - 3 | lubricant |
| **Total mass** | **200** - **500.0 mg** | **100 %** | |

| **Sub-coating layer** | |
|---|---|
| **Component** | **[%]** |
| Coating system | 1-5 |

| **Component a) Active-coating layer (with RIVA)** | | |
|---|---|---|
| **Component** | **mg/tab.** | **[%]** |
| Rivaroxaban (API) | 2.5 | - |
| Coating system | qs | 3-8 |

| **Top-coating layer** | |
|---|---|
| **Component** | **[%]** |
| Coating system | 1-5 |

### Sample composition:

| **Component b) Acetylsalicylic acid, 50 mg, tablet core** | | |
|---|---|---|
| **Component** | **mg/tab.** | **[%]** |
| Acetylsalicylic acid | 50.0 | 11.2 |
| Cellulose microcrystalline | 230.0 | 51.7 |
| Starch, maize | 114.0 | 25.6 |
| Silica, colloidal anhydrous | 4.0 | 0.9 |
| Stearic acid | 2.0 | 0.45 |

| **Component a) Rivaroxaban, 2.5 mg, f-c layer** | | |
|---|---|---|
| Rivaroxaban | 2.5 | 0.55 |
| Coating system | 42.5 | 9.6 |

### MANUFACTURING

### 1. Core tablet with ASA (immediate release) - Direct Compression Technology

Drug product excipients (except of lubricant) are sieving and mixing. Active substance is sieving. The drug product components (API + excipients except of lubricant) are placed in suitable container and blend until homogenous mixture is obtained. Lubricant is sieved into the mixture and blend to obtain tableting mass. The tableting mass is compressed using a tablet press to obtain tablets with the desired strength.

### 2. Layer with RIVA (immediate release) - Film-coating Technology

The film-coating system is suspended in medium and until homogenous suspension is obtained. The suspension is divided into 3-parts. One of them is used for sub-coating process. The amount of sub-coat is 1 - 5% of core tablets weight gain. The specified amounts of active substance (rivaroxaban) is added to the second part of film-coating suspension and mix until homogenous mixture is obtained. It is possible to carry out the homogenization step for suspension with API. It is possible to dilute the suspension with API. The amount of API-coat is 1 - 5% of core tablets weight gain. The third part of film-coating suspension is used for top-coating. The amount of top-coat is 1 - 5% of core tablets weight gain.

### III. BILAYER TABLET

Drug product in form of bi-layer tablet with acetylsalicylic acid in strength 50 mg and rivaroxaban in strength 2.5 mg. Drug product formulation components:

| **Drug product form with acetylsalicylic acid in strength 50 mg** | | | |
|---|---|---|---|
| **Component b) of ASA 50 mg** | **Function** | **Alternative type of the com ponent** | **Different components** |
| Acetylsalicylic acid | active substance | - | - |
| Cellulose microcrystalline (type 102) (mean particle size: 100 µm) | filler | Cellulose (mean particle size: 20 - 250 µm) | powdered cellulose, silicified microcrystalline cellulose |
| Starch, maize (native) (mean particle size: 10 - 15 µm) | filler, disintegrant | Starch (mean particle size: 5 - 45 µm) | potato starch, rice starch, pea starch, tapioca starch |
| Silica G, colloidal anhydrous (SSA 175 - 225 m²/g) | glidant | Hydrophilic fumed silica (SSA 75 - 410 m²/g) | hydrophobic fumed silica, fumed mixed oxides |
| Stearic acid (50) (stearic acid content: 40 - 60%) | lubricant | Stearic acid (stearic acid content: 40 - 100%) | palmitic acid, lauric acid, myristic acid |

| **Drug product form with rivaroxaban in strength 2.5 mg** | | | |
|---|---|---|---|
| **Component a) of RIVA 2.5 mg** | **Function** | **Alternative type of com ponents** | **Different components** |
| Rivaroxaban | active substance | - | - |
| Lactose monohydrate (mean particle size: about 40 µm) eventually (bulk density: 0.6 g/ml) | filler | Lactose (mean particle size: 15-140 µm) eventually (bulk density: 0.4-0.8 g/ml) | lactose anhydrous, lactose spray-dried, lactose inhalation |
| Cellulose microcrystalline (type 101)(mean particle size:50-70 µm) | filler | Cellulose (mean particle size: 20 - 250 µm) | powdered cellulose, silicified microcrystalline cellulose |
| Croscarmellose sodium (degree of substitution: 0.79) | disintegrant | Croscarmellose sodium (degree of substitution: - 0.85) | carboxymethylcellulose sodium,carboxymethylcellulose calcium |
| Hypromellose (E 5) (viscosity: 4-6 mPa s) | binder | Hypromellose (viscosity: 3-140 000 mPa s) | hydroxypropyl cellulose, hydroxyethyl cellulose, hydroxyethyl methyl cellulose |
| Sodium Lauryl sulphate (Texapon K-12) | solubilizer | | magnesium lauryl sulphate, cetyl alcohol, cetostearyl alcohol |
| Magnesium Stearate (specific surface area: 4-10 m²/g) | lubricant | Magnesium Stearate (specific surface area: 1 - 15 m²/g) | calcium stearate, zinc stearate, stearic acid, magnesium aluminium silicate |
| Purified Water ¹ | processing agent | | carbon dioxide free water, deaerated water, hard water, soft water, water for injection, water for irrigation |

| | | | |
|---|---|---|---|
| ¹ Purified water is processing agent using in granulation process and essentially removed during the manufacturing process. | | | |

### Drug product form (BILAYER TABLET)

Bilayer tablet:
- ASA 50 mg IR layer
- RIVA 2.5 mg IR layer

Bilayer tablet coated:
• ASA 50 mg IR layer
• RIVA 2.5 mg IR layer
• Film-coating layer

| **Component b) Acetylsalicylic acid, 50 mg, layer** | | |
|---|---|---|
| **Component** | **mg/tab.** | **[%]** |
| Acetylsalicylic acid | 50.000 | 35 - 70 |
| Cellulose microcrystalline | - | 10 - 90 |
| Starch, maize | - | 5-75 |
| Silica G, colloidal anhydrous | - | 0.1 - 5 |
| Stearic acid | - | 0.1 - 5 |
| **Total mass** | **70** - **200.0** | **100** |

| **Component a) Rivaroxaban, 2.5 mg, layer** | | |
|---|---|---|
| **Component** | **mg/tab.** | **[%]** |
| Rivaroxaban | 2.5 | 1.5 - 3.5 |
| Lactose monohydrate | - | 5-90 |
| Cellulose microcrystalline | - | 5-90 |
| Croscarmellose sodium | - | 0.5 - 25 |
| Hypromellose | - | 0.5 - 10 |
| Sodium Lauryl sulphate | - | 0.5 - 10 |
| Magnesium Stearate | - | 0.1 - 5 |
| **Total mass** | **80-140.0** | **100** |

### Sample composition:

| **Component b) Acetylsalicylic acid, 50 mg, IR layer** | | |
|---|---|---|
| **Component** | **mg/tab.** | **[%]** |
| Acetylsalicylic acid | 50.0 | 22.7 |
| Cellulose microcrystalline | 58.5 | 26.6 |
| Starch, maize | 29.4 | 13.4 |
| Silica G, colloidal anhydrous | 1.4 | 0.6 |
| Stearic acid | 0.7 | 0.4 |

| **Component a) Rivaroxaban, 2.5 mg, IR layer** | | |
|---|---|---|
| Rivaroxaban | 2.5 | 1.1 |
| Lactose monohydrate | 25.0 | 11.4 |
| Cellulose microcrystalline | 44.5 | 20.2 |
| Croscarmellose sodium | 4.0 | 1.8 |
| **Hypromellose** | 1.6 | 0.7 |
| Sodium Lauryl sulphate | 1.6 | 0.7 |
| Magnesium Stearate | 0.8 | 0.4 |

### MANUFACTURING

### 1. Layer with ASA (immediate release) - Direct Compression Technology

Drug product excipients (except of lubricant) are sieving and mixing. Active substance is sieving. The drug product components (API + excipients except of lubricant) are placed in suitable container and blend until homogenous mixture is obtained. Lubricant is sieved into the mixture and blend to obtain tableting mass.

### 2. Layer with RIVA (immediate release) - Wet Granulation Technology

The specified amounts of active substance (rivaroxaban) and intra-granular excipients are mixed with the aid of high-shear granulator to obtain the powder blend. The binder solution is sprayed on the powder blend during mixing in high-shear granulator. Then the main granulation phase is performed. The wet mass is transferred into the fluid bed dryer and the drying process is performed. The dried mass is passed through a screen into a suitable container. Lubricant is sieved into the mixture and blend to obtain tableting mass.

### 3. Tableting

The tableting mass with ASA and tableting mass with RIVA are compressed using a tablet press to obtain bilayer tablets with the desired strengths. The bilayer tablets may be film-coated.

### IV. MULTILAYERED TABLET

### Rivaroxaban / acetylsalicylic acid; multi-coated tablets, 2.5/50 mg

| **Component b) Acetylsalicylic acid, 50 mg, tablet IR** (immediate release) | | | |
|---|---|---|---|
| **Component** | **mg/tab.** | **[%]** | **Function** |
| Acetylsalicylic acid | 50.0 | 10-25 | active substance |
| Cellulose microcrystalline | | 10-90 | filler |
| Starch, maize | | 3-75 | filler, disintegrant |
| Silica G, colloidal anhydrous | | 0.1 - 1 | glidant |
| Stearic acid | | 0.1-3 | lubricant |
| **Total mass** | **200** - **500.0** | **100** | |

| **Sub-coating layer** | |
|---|---|
| **Component** | **[%]** |
| Coating system | 1-5 |

| **Component a) Active-coating layer (with RIVA)** | | |
|---|---|---|
| **Component** | **mg/tab.** | **[%]** |
| Rivaroxaban (API) | 2.5 | - |
| Coating system | qs | 3-8 |

| **Top-coating layer** | |
|---|---|
| **Component** | **[%]** |
| Coating system | 1-5 |

| | | |
|---|---|---|
| Multi-coated tablet | | |

| **Component** | **mg/tabl.** | **[%]** |
|---|---|---|
| Component b) ASA, 50 mg IR (core tablet) | 200 - 500 | - |
| Sub-coating layer | - | 1-5 |
| Component a) RIVA, 2.5 mg layer | - | 3-8 |
| Top-coating layer | - | 1-5 |

### MANUFACTURING

i.Granulate with ASA - mixing
ii.Core tablet with ASA, 50 mg - tableting
iii.Suspension with RIVA, 2.5 mg - mixing
iv.Suspension without API - mixing
v.Film coating - 3-steps (sub-coating, active coating, top-coating)

Coating system is based on PVA (polyvinyl alcohol) or HPMC (Hypromellose) or HPS (hydroxypropyl pea starch).

### Dissolution studies

The pharmaceutical compositions according to the present invention and the marketed referent formulations of Rivaroxaban-Xarelto 2.5 mg and Acetylsalicylic acid-Godamed 50 mg will subject to dissolution tests carried out in accordance with the test methods described in tables below:

**Table. Dissolution testing parameters - proposal 1**

| Parameter | Dissolution testing conditions for Rivaroxaban | Dissolution testing conditions for Acetylsalicylic acid |
|---|---|---|
| Medium: | Acetate buffer pH 4.5 | Acetate buffer pH 4.5 |
| Volume [ml]: | 900 | 900 |
| Apparatus type: | Apparatus type: 1 (baskets, according to European Pharmacopoeia 10^{th} edition) | Apparatus type: 1 (baskets, according to European Pharmacopoeia 10^{th} edition) |
| Temperature | 37°C± 0.5°C | 37°C± 0.5°C |
| RPM: | 100 | 100 |
| Time points [min]: | 10, 15, 20, 30, 45, 60, infinity test | 10, 15, 20, 30, 45, 60, infinity test |

**Table. Dissolution testing parameters - proposal 2**

| Parameter | Dissolution testing conditions for Rivaroxaban | Dissolution testing conditions for Acetylsalicylic acid |
|---|---|---|
| Medium: | Acetate buffer pH 4.5 | Acetate buffer pH 4.5 |
| Volume [ml]: | 900 | 900 |
| Apparatus type: | Apparatus type: 2 (paddles with sinkers, according to European Pharmacopoeia 10^{th} edition) | Apparatus type: 2 (paddles with sinkers, according to European Pharmacopoeia 10^{th} edition) |
| Temperature | 37°C± 0.5°C | 37°C± 0.5°C |
| RPM: | 75 | 75 |
| Time points [min]: | 10, 15, 20, 30, 45, 60, infinity test | 10, 15, 20, 30, 45, 60, infinity test |

The quantities of drug substance released from the tested products will be determined by HPLC. The mean value will be calculated from 6 tablets. Requirements are presented in table below.

**Table. Requirements of dissolution test for Rivaroxaban +Acetylsalicylic acid, 2.5 mg + 50 mg**

| | REQUIREMENTS |
|---|---|
| Dissolution of Rivaroxaban | After 30 minutes: |
| | for each tablet ≥ 85% |
| | (S₁= Q + 5; Q = 80%) |
| | The additional test is acceptable |
| Dissolution of Acetylsalicylic acid | After 15 minutes: |
| | for each tablet ≥ 85% |
| | (S₁= Q + 5; Q = 80%) |
| | The additional test is acceptable |

### Stability studies

Medicines must comply with the requirements of the chemical purity immediately after preparation (comply with the limits indicated in the release specification of the drug product), and within the period set by the relevant guidelines in time and storage conditions (comply with the limits indicated in the shelf-life specification of the drug product).

The requirements for related substances will be established according to guidelines CPCP/ICH/367/96 (ICH Q6A), and CPMP/ICH/2738/99 (ICH Q3B (R2)). The in-house chromatographic method for related substances will be selective for degradation products for Rivaroxaban and Acetylsalicylic acid according to Pharmacopoeia. Impurities of Rivaroxaban are specified in Pharmacopoeia No 01/2021:2932. Impurities of Acetylsalicylic acid are specified in Pharmacopoeia No 01/2017:0309. Stability studies of the invention, packed in foil blister and carton box will be carried out at conditions (25C/60%RH and 40C/75% RH).

**Table. Requirements for Related substances**

| Parameter | Rivaroxaban + Acetylsalicylic acid, 2.5 mg + 50 mg |
|---|---|
| | Requirements ¹ |
| **Chromatographic purity** | |
| Rivaroxaban | |
| - known impurity of RVXB | not more than 1.0% |
| - max single unspecified impurity RVXB | not more than 0.4% |
| - total impurities | not more than 1.5% |
| Acetvlsalicvlic acid | |
| - Salicylic acid | not more than 3.0% |
| - max single impurity Acetylsalicylic acid | not more than 0.2% |
| - total impurities (excluding salicylic acid) | not more than 1.0% |

| | |
|---|---|
| ¹The requirements were established according to guidelines CPMP/ICH/2738/99 (ICH Q3B (R2). The limit for Salicylic acid is in line with requirements of Acetylsalicylic acid tablets monographs in British Pharmacopoeia. | |

Assay of the active substances - Rivaroxaban and Acetylsalicylic acid in the specification for drug product Rivaroxaban / Acetylsalicylic acid 2.5 mg/50 mg will be provided by the HPLC method. Requirements will be established according to guidelines ICH Q6A and 3AQ11a. The acceptance criterion will be established as the range 95.0 ÷ 105.0 % of the claimed value.

### PK and bioequivalence studies

The fixed dose combination (FDC) of rivaroxaban and Acetylsalicylic acid (2.5 mg + 50 mg) is a substitution therapy in coronary artery disease (CAD), symptomatic peripheral artery disease (PAD) and patients after an acute coronary syndrome (ACS). Both active substances are orally administered anticoagulants, but the mechanism of action is different. Rivaroxaban is a selective direct factor Xa inhibitor but no effects on platelets have been demonstrated (Samama M. M. (2011). Thrombosis research, 127(6), 497-504). Acetylsalicylic acid is an antiplatelet agent irreversibly inhibiting PGH2 synthase (COX)-mediated formation of thromboxane A2 (TxA2) which in turns leads to inhibition of platelet aggregation. TxA2 is rapidly hydrolyzed non-enzymically to TxB2, the concentrations of which can be quantified in plasma and used as an index of TxA2 production (Warner, T. D., British journal of clinical pharmacology, 72(4), 619-633.). Synergistic pharmacological effect of administration of rivaroxaban and Acetylsalicylic acid was shown in phase III clinical trials VOYAGER PAD, ATLAS ACS 2-TIMI 51 and especially COMPASS study, where it was shown that patients assigned to rivaroxaban (2.5 mg twice daily) plus Acetylsalicylic acid (100 mg once daily) had better cardiovascular outcomes than those assigned to Acetylsalicylic acid alone and difference between rivaroxaban alone (5 mg) and Acetylsalicylic acid alone was not statistically significant (Bonaca, (2020). The New England journal of medicine, 382(21), 1994-2004; Eikelboom, The New England journal of medicine, 377(14), 1319-1330; Mega, J. L., The New England journal of medicine, 366(1), 9-19).

Bridging studies compare pharmacokinetic data between proposed FDC rivaroxaban/Acetylsalicylic acid (2.5 mg + 50 mg) vs rivaroxaban (2.5 mg) and Acetylsalicylic acid (50 mg) alone (bioequivalence study).

The purpose of establishing bioequivalence is to demonstrate equivalence in biopharmaceutics quality between the test investigational medicinal product (test IMP) and reference medicinal products.

Two medicinal products containing the same active substance are considered bioequivalent if they are pharmaceutically equivalent or pharmaceutical alternatives and their bioavailabilities (rate and extent) after administration in the same molar dose lie within acceptable predefined limits. These limits are set to ensure comparable *in vivo* performance, i.e., similarity in terms of safety and efficacy (CHMP Guideline on the Investigation of Bioequivalence, CPMP/EWP/QWP/1401/98 Rev. 1/Corr ^{∗∗}, London, January 2010).

To demonstrate the bioequivalence of a FDC medicinal product of rivaroxaban and Acetylsalicylic acid (test IMP) versus two co-administered reference IMPs, one containing rivaroxaban (Xalerto 2,5 mg film-coated tablets) and the other containing Acetylsalicylic acid (Godamed 50 mg tablets) the bioequivalence study are performed under fed conditions.

This study is designed as a single-dose, open-label, laboratory-blind, randomized, two-period, twotreatment, two-sequence, crossover bioequivalence study under fed conditions, with blood sampling period of 24 hours after dosing and with at least 7 days of washout between IMP administrations in Study Period 1 and 2.

Approximately 60 subjects are planned for enrolment and dosing. Blood samples will be collected at approximately 19 time point for rivaroxaban and Acetylsalicylic acid and 25 time point for SA. Concentrations of rivaroxaban, Acetylsalicylic acid and SA in plasma will be determined by validated LC-MS/MS methods. The pharmacokinetic parameters will be derived individually for each subject from rivaroxaban, Acetylsalicylic acid and SA concentrations in plasma.

The pharmacokinetic parameters AUC (area under the plasma concentration-time curve) and Cmax (maximum plasma concentration) will be statistically evaluated to assess the bioequivalence. Bioequivalence of the Test IMP and the Reference IMPs will be assessed on basis of 90% confidence intervals of geometric mean ratios T/R for the primary pharmacokinetic parameters AUC and Cmax of rivaroxaban, Acetylsalicylic acid and SA. Other pharmacokinetic parameters will be presented as secondary parameters.

According to the Guideline on the Investigation of Bioequivalence will be applied the bioequivalence acceptance range 80.00%-125.00% for the primary parameters.

The primary objective of the bioequivalence study is to evaluate the pharmacokinetic properties and to compare the bioavailability of the FDC medicinal product (test IMP) versus the reference IMPs in healthy volunteers under fed conditions.

The secondary study objective is to evaluate the safety of the test and reference IMPs. The adverse events and clinically relevant deviations from clinical laboratory tests, physical examinations, ECG and vital signs are followed for the evaluation of safety and its comparison between test and reference IMPs. Importantly, administration of Acetylsalicylic acid in FDC medicinal product developed by Adamed has a different dose interval than Acetylsalicylic acid used in COMPASS and VOYAGER PAD studies. In proposed FDC product Acetylsalicylic acid will be administered 50 mg twice daily, whereas in abovementioned phase III studies Acetylsalicylic acid was administered 100 mg once daily, as standard Acetylsalicylic acid therapy. Acetylsalicylic acid Therapeutic Equivalence study.

The primary objective of this phase III, open-label, cross-over, multicentre, therapeutic equivalence study was to evaluate equivalence in inhibiting platelet aggregation between twice-daily acetylsalicylic acid (IR) tablets 50 mg compared to once-daily acetylsalicylic acid enteric coated (EC) tablets 100 mg in adult patients after Acute Coronary Syndrome (ACS) or Coronary Artery Disease (CAD) or Symptomatic Peripheral Artery Disease (PAD). Approximately 100 patients will be consecutively treated with 2 regimens of Acetylsalicylic acid: (1) acetylsalicylic acid enteric coated tablets 100 mg administered once daily in the morning (8.00-9.00 AM) and (2) acetylsalicylic acid tablets 50 mg administered twice daily: in the morning (8.00-9.00 AM) then in the evening (8.00-9.00 PM). All intake regimens will be maintained for 10 consecutive days. Blood samples for platelet function assessment will be collected at 8.00 AM before IMP intake on day 1, day 11 (change of regimens) and day 21 (end of treatment). As the maximum life-span platelets is 8-10 days, study will not incorporate a wash-out period in between regimens.

The primary endpoint of the study is the change in platelet aggregation measured by VerifyNow^{®} Acetylsalicylic acid Test (Accumetrics, CA, USA). The VerifyNow^{®} system is a whole-blood, point-of-care assay that has been specifically designed for the monitoring of the inhibitory effects of Acetylsalicylic acid therapy. After the citrated tube is inserted into the cartridge, whole blood is mixed with the platelet agonists (arachidonic acid) and the fibrinogen-coated beads. Arachidonic acid is converted by the COX-1 enzyme (the molecular target of Acetylsalicylic acid therapy) into TxA2. When Acetylsalicylic acid does not completely inhibit COX-1-activity, TxA2 formation leads to platelet activation with subsequent microbead agglutination. The beads fall out of the solution and the increase in light transmittance through the sample is reported as Acetylsalicylic acid-Reaction Units (ARU) (van Werkum, J. W., Platelets, 19(7), 479-488). In comparison to the "gold" standard and most widely accepted platelet function tests (i.e., light transmittance aggregometry) the VerifyNow^{®} system is a simple point-of-care, fully automated, standardized test with high reproducibility (Tyagi, T., Nat Cardiovasc Res 1, 223-237). Due to the automated procedure, it is less operator-dependent and may decrease intra-laboratory variability (Gremmel, T., Platelet. Laboratory Monitoring of Antiplatelet Therapy). Secondary endpoints include: (1) identification of patients with Acetylsalicylic acid resistance based on VerifyNow^{®} Acetylsalicylic acid Test specific cut-off values as per manufacturers specification and (2) identification of patients with high bleeding risk with standard laboratory tests i.e. prothrombin time (PT), activated partial thromboplastin time (APTT), antithrombin III (AT III), fibrinogen.

## Claims

1. A fixed-dose pharmaceutical composition for oral administration is provided, the composition comprising:
a) 2.5 mg of rivaroxaban and one or more pharmaceutically acceptable excipients; and
b) 50 mg of Acetylsalicylic acid and one or more pharmaceutically acceptable excipients, and
one or more pharmaceutically acceptable excipients.

2. The pharmaceutical composition according to (1), wherein the pharmaceutical composition is a single dosage form in the form of a tablet, in which the component a) forms a first layer, the component b) forms a second layer, and the first layer at least partially covers the surface of the second layer. Preferably, the tablet is a bilayer tablet.

3. The pharmaceutical composition according to (1) or (2), wherein the pharmaceutical composition is a single dosage form in the form of a tablet, in which the component a) forms a first layer, the component b) forms a second layer, and the first layer completely covers the surface of the second layer. Preferably, the tablet is a bilayer tablet. In this aspect the component b) can form a first layer while the component a) forms a second layer.

4. The pharmaceutical composition according to (3), wherein the tablet is uncoated or coated.

5. The pharmaceutical composition according to (1), wherein the pharmaceutical composition is a single dosage form in the form of a coated tablet, in which the component b) forms a core of the tablet and component a) is in a form of a coat applied on this core.

6. The pharmaceutical composition according to (5), wherein further coat is applied on the coat formed by component a).

7. The pharmaceutical composition according to (5) or (6), wherein an intermediate coat is applied on the component b), between the tablet core and the component a).

8. The pharmaceutical composition according to (1), wherein the pharmaceutical composition is a single dosage form in the form of a hard capsule, in which the component a) is in a form of a granulate, a pellet or a tablet, preferably a tablet or a coated tablet and component b) is in a form of a granulate, a pellet or a tablet, preferably a tablet or a coated tablet.

9. The pharmaceutical composition according to any one of claims 1 to 8, wherein the component a) comprises, as the at least one excipient, at least one of a filler, a disintegrant, a binder, a lubricant, a glidant and any combination thereof, preferably, the component a) comprises 5 to 90 wt.% of a filler, 0.5 to 25 wt.% of a disintegrant, 0.1 to 5.0 wt.% of a lubricant, 0.4 to 10 wt.% of a binder, and optionally 0.1 to 5.0 wt.% of a glidant based on the total weight of the component a).

10. The pharmaceutical composition according to any one of claims 1 to 9, wherein the component b) comprises, as the at least one excipient, at least one of a filler, a disintegrant, a lubricant, a glidant and any combination thereof, preferably, the component b) comprises 5 to 90 wt.% of a filler, 0.3 to 5 wt.% of a disintegrant, 0.1 to 5.0 wt.% of a lubricant, 0.1 to 5.0 wt.% of a glidant, and optionally 0.4 to 5 wt.% of a binder, based on the total weight of component b).

11. The pharmaceutical composition according to any one of claims 1 to 10, wherein the pharmaceutical composition has an in vitro dissolution rate of rivaroxaban or Acetylsalicylic acid of 85 %, based on the total rivaroxaban or Acetylsalicylic acid content, respectively, of the single dosage form, as determined by using European Pharmacopoeia edition 10 Apparatus Baskets "1" in 900 mL volume of Acetate buffer pH 4.5, 37°C ± 0.5°C, 100 RPM or Apparatus type: 2 paddles with sinkers in 900 mL volume of Acetate buffer pH 4.5, 37°C ± 0.5°C, 75 RPM

12. A pharmaceutical composition as defined in any one of claims 1 to 11 for use in a prevention of atherothrombotic events in adult patients after an acute coronary syndrome (ACS) with elevated cardiac biomarkers or for the prevention of atherothrombotic events in adult patients with coronary artery disease (CAD) and/or symptomatic peripheral artery disease (PAD) at high risk of ischaemic events.

13. The pharmaceutical composition for use according to claim 12, wherein the pharmaceutical composition is administered orally two times daily, and preferably 11 to 13 hours apart, and more preferably 12 hours apart.

14. The pharmaceutical composition for use according to claim 12 or 13, wherein the pharmaceutical composition is a single dosage form, which is administered orally two times 12 hours apart.

15. The pharmaceutical composition for use according to any one of claims 12 to 14, wherein pharmaceutical composition is a single dosage form and provides, after oral administration, a maximum plasma concentration of rivaroxaban (Cₘₐₓ) of 77.69±28.2 ng/mL after median tₘₐₓ of 2 hours after administration, and an area under the plasma concentration time curve from time zero to infinity of 481.26±22.3 ng·h/mL, as determined by human PK studies.

16. The pharmaceutical composition for use according to any one of claims 12 to 15, wherein the pharmaceutical composition provides, after oral administration of a single dosage form of the pharmaceutical composition twice a day and preferably 12 hours apart, a maximum plasma concentration in steady state (Cmax) of Acetylsalicylic acid of 328.25±5.69 ng/mL after median tmax of 0.83 h and an area under the plasma concentration-time curve from time zero to infinity in steady state (AUC) of 531.75±2.80 ng·h/mL, as determined by human PK studies..

17. Combination of 2.5 mg of rivaroxaban and 50 mg of acetylsalicylic acid for use in a therapy of reducing the risk of myocardial infarction, stroke or cardiovascular death in a human patient with coronary artery disease and/or peripheral artery disease, comprising the dosage regimen of administering orally to the human patient twice daily, preferably 11 to 13 hours apart.

18. The combination according to claim 17, wherein the dosage regimen of administering is 12 hours apart.

19. The combination according to claim 17 or 18, wherein 2.5 mg of rivaroxaban and 50 mg of acetylsalicylic acid fixed are in a form of a fixed-dose pharmaceutical composition.
